# EUROPEAN PATENT SPECIFICATION

(11) **EP 0 668 290 B1**
(45) Date of publication and mention of the grant of the patent: **11.11.1998**
(21) Application number: 95105215.8
(22) Date of filing: 27.02.1991
(51) Int. Cl.: C07H 19/04, C07D 207/273, A61K 31/70, A61K 31/40

(54) **Physiologically active substance well capable of permeating biomembrane**
Physiologisch aktive Substanz die fähig ist Biomembran zu permeieren
Substance-active physiologiquement capable de traverser une biomembrane

(30) Priority: 28.02.1990 JP 50116/90
(43) Date of publication of application: 23.08.1995
(62) Divisional of application: 91905340.5
(73) Proprietor: TEIKOKU SEIYAKU KABUSHIKI KAISHA, Okawa-gun Kagawa-ken (JP)
(72) Inventor: Kitagawa, Kouki, Itano-gun, Tokushima 771-02 (JP); Hibi, Toru, Kawasaki-shi, Kanagawa 213 (JP); Mizobuchi, Noriko, Okawa-gun, Kagawa 769-26 (JP)
(74) Representative: Tiedtke, Harro, Dipl.-Ing.

(56) References cited:
- EP-A- 0 187 052
- EP-A- 0 257 378
- DATABASE WPI Week 7622, 1976 Derwent Publications Ltd., London, GB; AN 76-4056X & JP-A-51 043 769 (ASAHI CHEMICAL IND. K.K.) 15 April 1976
- DATABASE WPI Week 8104, 1981 Derwent Publications Ltd., London, GB; AN 81-04915D & JP-A-55 149 299 (YAMASA SHOYU K.K.) 21 November 1981

## Description

### Specification

### Technical Field

The present invention concerns a physiologically active substance having high biomembrane permeability, more specifically, blood-brain barrier (BBB) permeability. Since the physiologically active substance according to the present invention has a high blood-brain barrier permeability, it can provide a sufficient intraventricular physiological activity even by means of subcutaneous administration or intravenous injection which is simpler than intraventricular administration.

### Background Art

The present invention specifically relates to physiologically active substances which are based on an adamantane derivative of either a nucleoside compound or a pyrrolidone compound.

JP-A-55-149 299 discloses adamantane linked at an N-6 position of codycepin via an amide bond. This substance having an adamantane linked to the purine moiety of the nucleoside residue is intended to have antibacterial and anti-tumor activity and to inhibit adenyl-deaminase activity.

EP-A-0 257 378 discloses a compound as an anti-viral agent, the compound having an adamantane linked via an amide group and a lower alkylene linker group to the CH₂ group of a 5'-deoxy-ribose ring of a pyrimidine nucleoside derivative.

JP-A-51-0 437 69 discloses adamantane derivatives bound to nucleosides - namely 1-β-D-arabinofuranosyl cytosine derivatives - at the N-4 position of the pyrimidine ring. These compounds are said to be useful in treating leukemia and solid tumors.

Further, EP-A-0 187 052 discloses adamantyl-pyrrolidone conjugates, wherein the adamantyl group is linked via an amide group to the 3-C-atom of the pyrrolidone ring system.

The present inventors have tried to improve the BBB permeability, which has not yet been studied by so much. Further, it has been expected that if the BBB permeability of enkephalins as a Reference Example can be improved, the BBB permeability of other substances with poor BBB permeability can also be increased to extend the application region.

### Disclosure of the Invention

The physiologically active substance well capable of permeating biomembrane according to the present invention has a feature in that it is represented by the general formula: 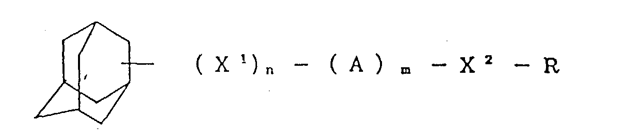 where
X¹ and X², which may be identical with or different from each other, represent an ether bond, urethane bond, ester bond or amide bond, A represents a lower alkylene group in which n = 0 if m = 0,
n = 0 or 1 if m = 1,
R represents a 5'-O -nucleoside compound or N-linked 2-pyrrolidone.

As has been described above, adamantyl groups are introduced to the physiologically active substance according to the present invention, so that the poor BBB permeability of the physiologically active substance can be improved. Accordingly, it has been shown such a possibility as capable of administering, by way of hypodermic injection, the above specified physiologically active susbtances which have not hitherto shown effects in the brain unless directly administered intraventricularly.

In the present invention, optionally one or more positions in the adamantyl group may be substituted with a group for enhancing the BBB permeability of the physiologically active substance. As specific groups, there can be mentioned, lower alkyl group such as methyl group or ethyl group, hydroxyl group, lower alkoxy group, amino group, carboxyl group and carboxy lower alkyl group. It is, however, desirable that the compound is not so much hydrophilic.

According to the present invention, the BBB permeability can be enhanced by introducing the adamantyl group to a substance of poor BBB permeability to provide a possibility that a nucleoside compound or 2-pyrrolidone show effetcs in the brain without being directly administered intraventricularly.

### BRIEF DESCRIPTION OF THE DRAWINGS

Fig. 1 is a view illustrating the structural formula of an enkephalin derivative (proto type a-d) as a Reference Example;
Fig. 2 is a graph illustrating the result of measurement for the in vivo analgesic activity of the derivative a as a Reference Example;
Fig. 3 is a graph illustrating the result of measurement for the in vivo analgesic activity of the derivative b as a Reference Example;
Fig. 4 is a graph illustrating the result of measurement for in vivo analgesic activity of morphine hydrogen chloride as a Reference Example;
Fig. 5 is a graph illustrating the result of measurement for in vivo analgesic activity of the control as a Reference Example.

### BEST MODE FOR PRACTICING THE INVENTION

The present inventors have made various studies for improving the BBB permeability of the enkephalin derivative as a Reference Example and, as a step therefor, have tried to introduce an adamantyl group for improving the fat solubility.

As a proto type before introducing the adamantyl group, a derivative : H-Tyr-D.Ala-Gly-Phe-Leu-OH in which the second Gly from the N-terminus of enkephalin is substituted with D-Ala was used as a Reference Example. For the above-mentioned derivative, it has already been reported that the derivative has a high resistivity to an aminopeptidase and has enhanced analgesic effect.

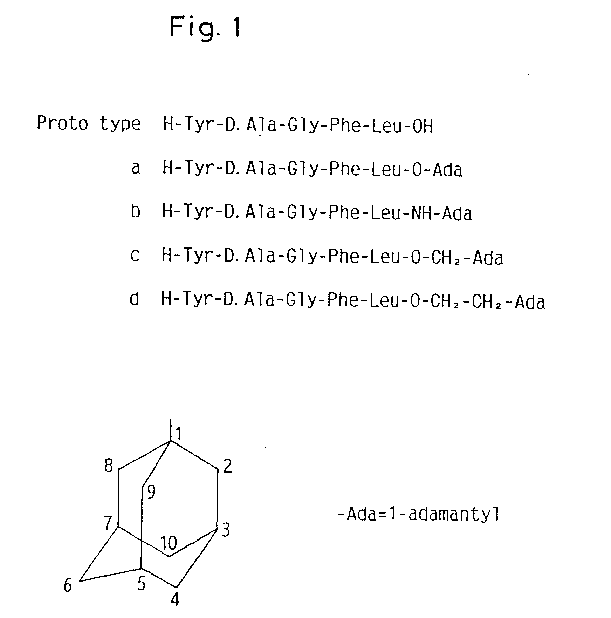

The four kinds of derivatives a to d shown in Fig. 1, in which the adamantyl group is introduced to the derivative, were synthesized as further Reference Examples, in which Ada represents an adamantane residue.

The synthesis of the Reference Example derivatives a to d is described in EP-B1-0 516 851 which represents the parent patent specification relating to the present Divisional Application. gradient elution using 5% acetic acid (200 ml) and acetonitrile (200 ml). The main fraction was concentrated and freeze-dried to obtain white feather-like crystals.
Yield: 110 mg (50 %), Melting point : 117 - 122°C
Rf₁: 0.28 [α]_{D} = +34.9° (C=0.2, DMF)
FAB-MS: 732.0 [M+H]⁺

| Elemental analysis: C₄₁H₅₇N₅O₇·1.5H₂O | | | |
|---|---|---|---|
| Calculated value | C 64.88, | H 7.97, | N 9.23 |
| Measured value | C 64.61, | H 7.72, | N 8.91 |

### Reference Example

Adamantylated enkephalin derivatives a and b were examined for in vivo analgesic effect and in vitro suppressing effect to shrinkage of extirpated intestine of guinea pig by electric stimula- tions.

### Measurement for in vivo analgesic activity

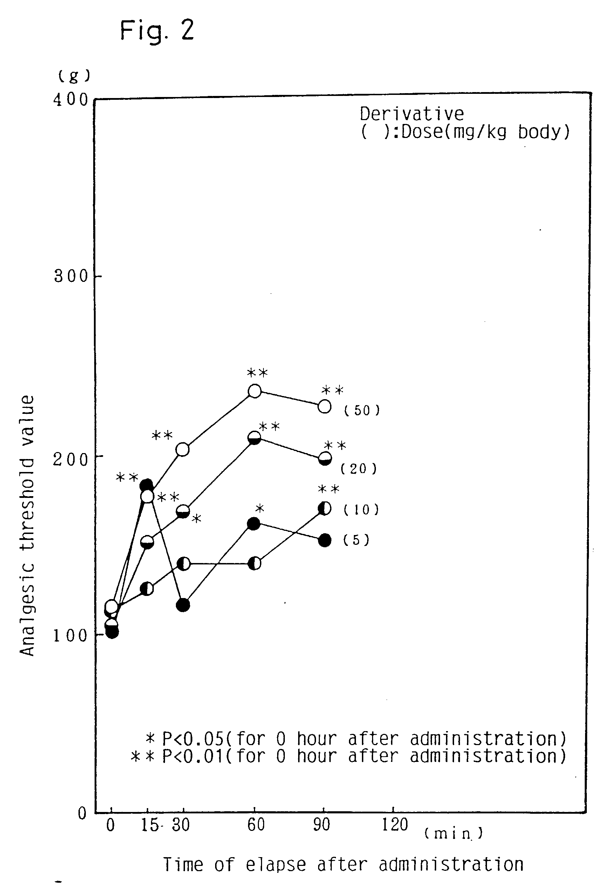

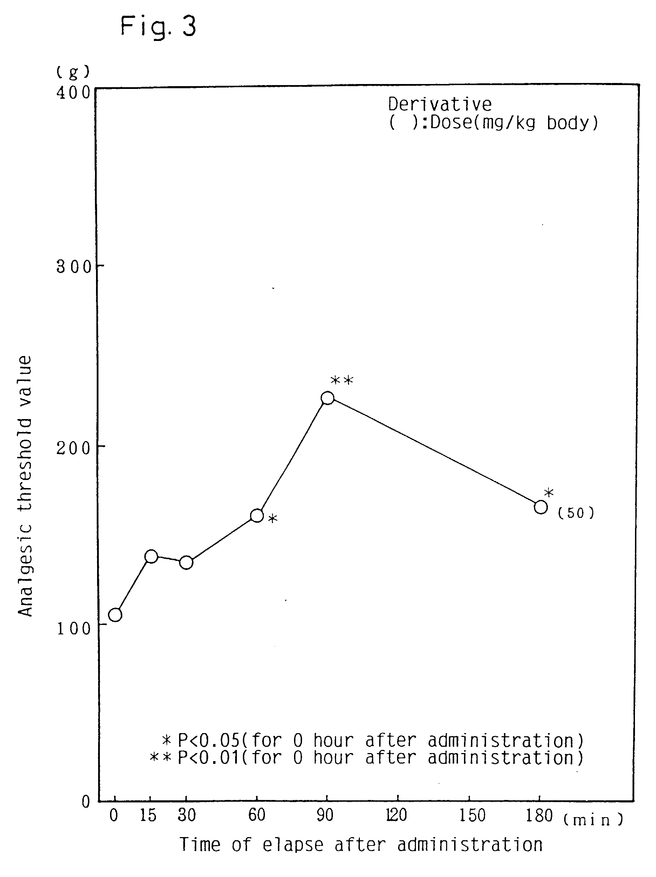

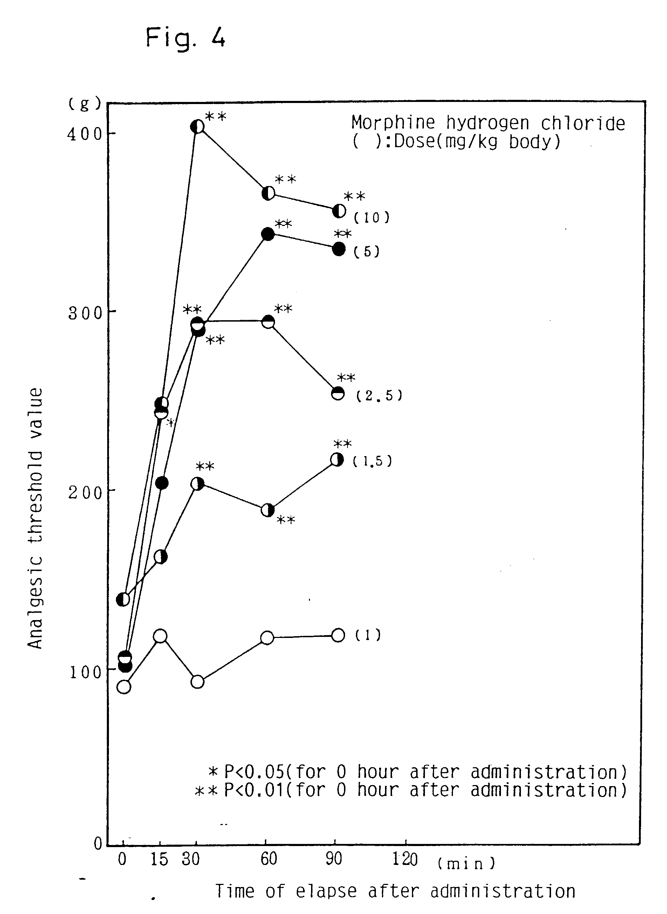

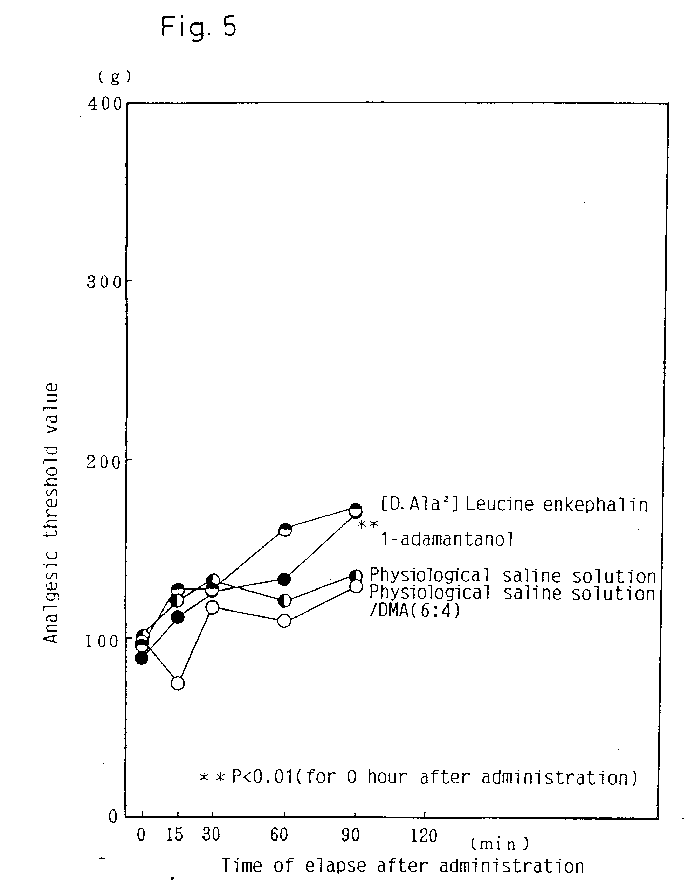

Adamantylated enkephalin derivatives a and b are subcutaneously administrated to a mouse, and an analgesic effect in the central nervous system was measured by Randall Selitto method. That is, the derivatives a and b were dissolved in a mixed solution of DMA : physiological saline solution (4:6) and about 100 µl of the solution was subcutaneously administrated to a mouse (dd-Y series: about 20 g) (1 - 50 mg/kg), and the analgesic effect was measured on every predetermined time interval after the administration by the Randall Selitto method. Morphine hydrogen chloride was used as a comparative drug and [D-Ala²] leucine enkephalin, 1-adamantanol, physiological saline solution and mixed solvent of physiological saline solution + DMA (6:4) were used as controls, respectively. The test results of this Reference Example are shown in Fig. 2 (derivative a), Fig. 3 (derivative b), Fig. 4 (morphine hydrogen chloride) and Fig. 5 (control).

The derivatives a and b having adamantyl group introduced on the C-terminus showed analgesic activity inferior to morphine hydrogen chloride but superior to the controls.

### Measurement for in vitro analgesic activity

A suppressing effect for the shrinkage of extirpated intestine of guinea pig of the adamantylated enkephalin derivatives a and b as Reference Example compounds was measured in accordance with the method of Kosterlitz (H.W. Kosterlitz, A.A. Watterfield, Ann. Rev. Pharmacol., 15, 29, (1975)). Further, morphine hydrogen chloride was used as a comparative drug and [D-Ala²] leucine enkephalin was used as a control. The test results are shown in Table 1.

**Table 1**

| Compound | ED₅₀ (mol) (mean ± s.e.m.) | Relative Potency |
|---|---|---|
| Morphine hydrogen chloride | 3.2 ± 0.43 × 10⁻⁸ (n=17) | 1.00 |
| [D-Ala²] Leucine enkephalin | 3.6 ± 1.17 × 10⁻⁸ (n=7) | 0.89 |
| | | |
| Derivative a | 9.4 ± 4.82 × 10⁻⁸ (n=7) | 0.34 |
| Derivative b | 1.5 ± 0.71 × 10⁻⁸ (n=5) | 2.13 |

Each of the Reference Example derivatives a and b showed suppression for shrinkage each of which was antagonized by naloxon. That is, it can be seen that the derivatives have morphine-like activity irrespective of introduction of the adamantyl group.

From the test results of the Reference Example described above, it can be confirmed that the adamantylated enkephalins show morphine-like analgesic effect by hypodermic administration. This shows improvement in the BBB permeability and clinical application as a hypodermic injection solution is expected if the safety is confirmed.

From the results described above, it is shown that the BBB permeability is improved by the introduction of the adamantyl group while keeping the physiological activity and similar effects can also be expected to analogous derivatives. However, since it may be considered that the activity is lost depending on the position of introducing the adamantyl group in view of the nature of the physiological active substance, a sufficient care is necessary upon introduction.

Further, introduction of the adamantyl group by the following reaction was performed to zidovudine (azide thymidine, hereinafter simply referred to as AZT) as an anti-viral chemical therapeutical agent. 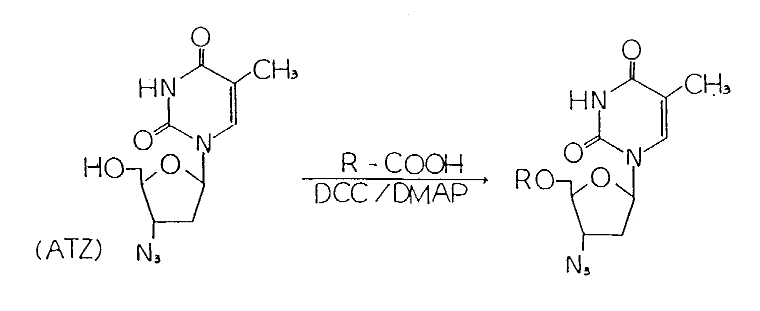 R used herein represents:

| | | |
|---|---|---|
| (1) | -CO-Ada | derivative e |
| (2) | -CO-CH₂-Ada | derivative f |
| (3) | -Gly-CO-Ada | derivative g |
| (4) | -β.Ala-CO-Ada | derivative h |
| (5) | -GABA-CO-Ada. | derivative i |

Description will now be made more specifically.

### Example 1

### Preparation of AZT-CO-Ada (derivative e)

Ada-COOH (135 mg, 0.8 mmol) were dissolved in DMF (10 ml), to which AZT (100 mg, 0.37 mmol), DCC (162 mg, 0.79 mmol) and 4,4-dimentylaminopyridine (69 mg, 0.56 mmol) were added under ice cooling and then stirred at 4°C for 48 hours. After filtering the resultant urea derivative, DMF was distilled off under a reduced pressure and residue was dissolved with addition of ethyl acetate and then washed with 5% solution of sodium hydrogen carbonate, an aqueous saturated solution of sodium chloride and water successively three times for each. After drying the ethyl acetate layer with sodium sulfate, the solvent was distilled off under reduced pressure, the residue was dissolved in n-hexane - ethyl acetate (7:3) (1 ml), and purified in silica gel column chromatography using the same solvent system as an eluting solution. Fractions containing the desired product were collected and, after distilling off the solvent under a reduced pressure, the residue was powderized with ether, collected by filtration and then dried.
Yield: 66 mg (41%), Melting point : 120 - 123°C
FT-IR: 2121 cm⁻¹ (-N₃)
FAB-MS: 430.2 [M+H]⁺, 452.2 [M+Na]⁺

| Elemental analysis: C₂₁H₂₇N₅O₅ | | | |
|---|---|---|---|
| Calculated value | C 58.73, | H 6.34, | N 16.31 |
| Analyzed value | C 59.12, | H 6.63, | N 16.04 |

### Example 2

### Preparation of AZT-CO-CH₂-Ada (derivative f)

The procedures were the same as those in Example 1 except for using Ada-CH₂COOH instead of Ada-COOH.
Yield: 79.4 mg (48 %), Melting point : 95 - 99°C
FT-IR: 2106 cm⁻¹ (-N₃)
FAB-MS: 444.2 [M+H]⁺, 466.2 [M+Na]⁺

| Elemental analysis: C₂₂H₂₉N₅O₅ | | | |
|---|---|---|---|
| Calculated value | C 59.60, | H 6.59, | N 15.79 |
| Analyzed value | C 59.74, | H 6.95, | N 15.31 |

### Example 3

### (1) Preparation of Ada-CO-NH-CH₂-COOH

Glycine (0.98 g, 13 mmol) was dissolved in 150 ml of water containing 4N sodium hydroxide (5 ml, 20 mmol), to which a 10 ml THF solution of 1-adamantane carbonylchloride (1.35 g, 6.5 mmol) was added for 30 min, and the reaction solution was vigorously stirred under ice cooling for 3 hours. After the reaction was over, the pH was adjusted to 7 with 1N hydrochloric acid, the THF was distilled off, the residue was dissolved in ethyl acetate, and the organic layer was washed successively, three times with 1N hydrochloric acid, three times with an aqueous saturated solution of sodium chloride and then three times with water. After drying the ethyl acetate layer with sodium sulfate, the solvent was distilled off and the residue was powderized with addition of ether.
Yield: 73 %, Melting point : 200 - 203°C

| Elemental analysis: C₁₃H₁₉NO₃.1/4H₂O | | | |
|---|---|---|---|
| Calculated value | C 64.59, | H 8.13, | N 5.80 |
| Analyzed value | C 64.64, | H 8.15, | N 5.74 |

### (2) Preparation of AZT-CO-CH₂-NH-CO-Ada (derivative g)

The procedures were the same as those in Example 1 except for using Ada-CO-Gly-OH instead of Ada-COOH, applying silica gel column chromatography as final purification by using chloroform → chloroform methanol (30:1) as an eluting solution and then powderizing with n-hexane.
Yield: 139.1 mg (76 %), Melting point : 95 - 98°C
FT-IR: 2109 cm⁻¹ (-N₃)
FAB-MS: 487.2 [M+H]⁺, 509.2 [M+Na]⁺

| Elemental analysis: C₂₃H₃₀N₆O₆.1/4H₂O | | | |
|---|---|---|---|
| Calculated value: | C 56.26, | H 6.26, | N 17.12 |
| Measured value : | C 56.71, | H 6.29, | N 16.62 |

### Example 4

### (1) Preparation of Ada-CO-β.Ala-OH

Procedures were the same as those in Example 3-(1) except for using β-alanine instead of glycine.
Yield: 85 %, Melting point : 187 - 192°C

| Elemental analysis: C₁₄H₂₁NO₃ | | | |
|---|---|---|---|
| Calculated value | C 66.90, | H 8.42, | N 5.57 |
| Measured value | C 66.82, | H 8.63, | N 5.32 |

### (2) Preparation of AZT-CO-(CH₂)₂-NH-CO-Ada (derivative h)

Procedures were the same as those in Example 3 except for using Ada-CO-β.Ala-OH instead of Ada-COOH and powderization with ether after chromatography.
Yield: 143.9 mg (77 %), Melting point : 92 - 95°C
FT-IR: 2108 cm⁻¹ (-N₃)
FAB-MS: 510.3 [M+H]⁺, 523.3 [M+Na]⁺

| Elemental analysis: C₂₄H₃₂N₆O₆.1/4H₂O | | | |
|---|---|---|---|
| Calculated value | C 57.07, | H 6.49, | N 16.64 |
| Measured value | C 57.39, | H 6.75, | N 16.12 |

Introduction of adamantyl group was attempted to 2-pyrrolidone which was expected as an antidementia drug.

### Example 5

2-pyrrolidone (850 mg) and DBU (1.53 g) were dissolved in methylene chloride (20 ml), to which a solution of adamantyl-1-carbonyl chloride (1.98 g) dissolved in methylene chloride (5 ml) was dropped under ice cooling and then left at a room temperature over one night and one day. After washing the organic solvent twice with 0.05N hydrochloric acid (40 ml), and twice with water (40 ml) successively, it was dried with sodium sulfate. The solvent was dried to solid under reduced pressure, subjected to column fractionation under the following conditions, the main fraction was separated, dissolved in methylene chloride and recrystallized from hexane to obtain white acicular crystals.
Yield: 400 mg (16%), Melting point: 86 - 86.5°C
Rf₅: 0.29
Column fractionation condition
Column : ø 3 cm x 35 cm
Filler : Kieselgel 60 (230 - 400 mesh ASTM)
Eluting solution: Methylene chloride

The compounds (Examples 1 - 5) synthesized as described above are introduced with adamantyl groups and it is expected that a preferred BBB permeability is provided to obtain a sufficient intraventricular activity.

## Claims

1. A substance represented by the general formula: 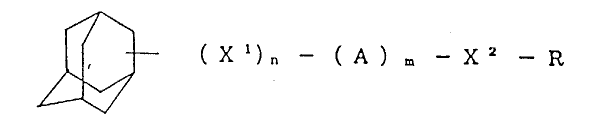 where
X¹ and X², which may be identical with or different from each other, represent an ether bond, urethane bond, ester bond or amide bond, A represents a lower alkylene group in which n = 0 if m = 0,
n = 0 or 1 if m = 1,
R represents a 5'-O -nucleoside compound or N-linked 2-pyrrolidone.

2. A substance as defined in claim 1,
wherein R represents zidovudine.

## Patentansprüche

1. Physiologisch aktive Substanz gemäß der allgemeinen Formel: 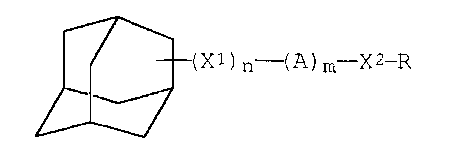 wobei X¹ und X² - die miteinander identisch oder voneinander verschieden sein können - eine Etherbindung, Urethanbindung, Esterbindung oder Amidbindung darstellen, A eine niedere Alkylengruppe darstellt, wobei n = 0 ist, wenn m = 0 ist, und n = 0 oder 1 ist, wenn m = 1 ist, R eine 5'-O-Nukleosid-Verbindung oder ein N-verknüpftes 2-Pyrrolidon darstellt.

2. Substanz nach Anspruch 1, wobei R Zidovudin darstellt.

## Revendications

1. Substance représentée par la formule générale : 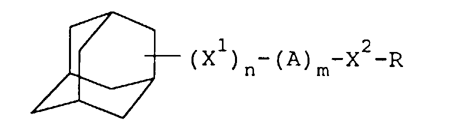 dans laquelle
X¹ et X², qui peuvent être identiques ou différents, représentent une liaison éther, une liaison uréthane, une liaison ester ou une liaison amide,
A représente un groupe alkylène inférieur,
n = 0 quand m = 0, et n = 0 ou 1 quand m = 1,
R représente un composé 5'-O-nucléoside ou une 2-pyrrolidone N-liée.

2. Substance suivant la revendication 1, dans laquelle R représente la zidovudine.
